# EUROPEAN PATENT APPLICATION

(11) **EP 1 205 556 A1**
(43) Date of publication of application: **15.05.2002**
(21) Application number: 00124623.0
(22) Date of filing: 10.11.2000
(51) Int. Cl.: C12P 7/02, C12P 7/26

(54) **Process for the preparation of trans-carveol**

(71) Applicant: AVENTIS ANIMAL NUTRITION S.A., 92164 Antony Cedex (FR)
(72) Inventor: Wouter, Adrian Duetz, 8049 Zürich-Hongg (CH); Witholt, Bernard, 8032 Zürich (CH); Jourdat, Catherine, 69110 Sainte Foy Les Lyon (FR)
(74) Representative: Mérigeault, Shona

(57) **Abstract**

A process for the production of *trans*-carveol which process comprises (a) incubating in the presence of D-limonene, a culture medium comprising microbial cells expressing an oxygenase capable of dioxygenation of toluene and/or napththalene and (b) recovering the *trans*-carveol from the culture medium.

## Description

The present invention relates to a process for the preparation of *trans*-carveol using a microbial cell culture expressing an oxygenase capable of dioxygenation of toluene and/or naphthalene to the corresponding dihydrodiols.

D-limonene is the main constituent of orange and lemon peel oil which is a by-product of the orange juice industry in quantities of approximately 50 000 tons per year. Its low price makes it an attractive starting compound for industrially relevant fine-chemicals and flavour compounds with identical carbon-skeletons, such as carveol, carvone, and perillyl alcohol. The regiospecific introduction of carbonyl or hydroxy groups by chemical catalysis, however, is difficult because the electronic properties of the allylic methylene groups (carbon 1 and 2) and the allylic methyl groups (carbon 5 and 6) are rather similar. For this reason, enzymatic oxidation has been considered as early as the 1960's and numerous D-limonene *trans*forming bacterial and plant cells have been reported on since.

Until now, the only biocatalysts showing an absolute regiospecificity were derived from plants. The P450 enzymes limonene 3-hydroxylase and limonene-6-hydroxylase (isolated from peppermint and spearmint microsomes) convert their natural substrate L-limonene and - at lower rates ― D-limonene to isopiperitenol and carveol respectively. Another example is the oxidation of D-limonene in the 6 position to *cis-* and *trans*-carveol and carvone by *Solanum aviculare* and *Dioscorea deltoidea*. The specific activities of these plant enzymes, however, are insufficient for industrial applications.

Microbial strains found so far to be capable of bioconversion of D-limonene generally yielded a mixture of oxidation products. Recent examples are the conversion of D-Limonene to -terpineol and 6-hydroxy-carveol by the honey fungus *Armillareira mellae*; to isopiperitenone, limonene-1,2 *trans* diol, *cis*-carveol and perillyl-alcohol, isopiperitenol, and -terpineol by *Aspergillus cellulosae*; to carveol -terpineol, perillyl alcohol and perillyl aldehyde by the D-limonene degrading strain *Bacillus stearothermophilus* BR388, and the same conversions by an *Escherichia coli* construct containing a plasmid with chromosomal inserts from this strain The most regiospecific microbial biocatalyst reported so far is the basidiomycete *Pleurotus sapidus* which converts D-limonene to *cis-* and *trans*-carveol and carvone, albeit at a relatively low rate.

In a number of studies, strains with the capability to use D-limonene as the sole source of carbon and energy were isolated and subsequently assessed for the accumulation of degradation pathway intermediates during growth. Most of these studies were inconclusive as mixtures of oxidation products were detected and it generally remained unclear whether these were genuine pathway intermediates or dead-end products resulting from an incomplete regiospecificity of the pathway enzymes. A recent, more successful, example is the work of Van der Werf and De Bont who proved that *Rhodococcus erythropolis* DCL14 degraded D-limonene via epoxidation at the 1,2 position and managed to clone the gene encoding the epoxidase in *Escherichia coli,* resulting in a biocatalyst for the high-yield production of D-limonene-1,2-epoxide.

US Patent No. 5688673 discloses a process for the production of a mixture of carveol, carvone, perillyl alcohol, and terpineol using a DNA segment of a *Bacillus stearothermophilus*.

We have now discovered a range of strains expressing oxygenases which are capable of producing *trans*-carveol from D-limonene.

According to the present invention, there is provided a process for the production of *trans*-carveol which comprises (a) incubating in the presence of D-limonene, a culture medium comprising microbial cells expressing an oxygenase which is capable of dioxygenation of toluene and/or napththalene, and (b) recovering the carveol from the culture medium.

The process of the present invention is the only known method to use bacteria that is uniquely suitable to provide not only pure *trans*-carveol, but also this product in significant yields from D-limonene. Furthermore, the process is relatively simple and economical, thus making it attractive for use in an industrial application.

The process of the present invention relates to a process for the production of *trans* carveol from D-limonene using microbial strains expressing oxygenases which are capable of dioxygenation of toluene and/or napththalene. The microbial strain may suitably be bacterial, fungal or yeast. Preferably, the strain is bacterial. Strains capable of dioxygenation of toluene and/or napththalene are defined as strains being capable of degrading toluene through a pathway which involves the dioxygenation of the aromatic ring as an initial degradation step, generally referred to as the 'tod' pathway.

The process comprises providing a cell culture comprising the cells expressing such oxygenases and incubating the cells in the presence of D-limonene to produce isolatable amounts of *trans*-carveol. Strains suitable for use in the present invention include strains from the genus *Rhodococcus*, for example *Rhodococcus opacus* and *Rhodococcus globerulus*, and from the genus *Pseudomonas*. The preferred strain is that from the genus *Rhodococcus* and in particular, a strain *Rhodococcus opacus* PWD4 (DSMZ 44313).

The gene encoding the oxygenases may suitable be cloned and expressed in a bacterial or yeast strain.

The strain expressing the oxygenase must be capable of accepting toluene and/or naphthalene, preferably be capable of dioxygenation of toluene and/or naphthalene to the corresponding dihydrodiols, toluene-cis-1,2-dihydrodiol and cis-1,2-dihydroxynaphthalene.

The incubation may suitably be carried out at a temperature of from 15 to 40°C, preferably from 20 to 30°C.

The D-limonene is preferably provided in the culture medium in the gas phase or as a second phase which is liquid, either pure or dissolved in an inert solvent such as hexadecane. Suitably, the D-limonene is added in small increments over time directly to the culture medium. Such procedures are well known in the art.

The growth medium used in the cell culture may be any suitable mineral media including a suitable carbon source in the presence of a suitable inducer such as toluene.

The *trans*-carveol product may be isolated from the culture medium by solid phase extraction or by extraction with a suitable organic solvent. When D-limonene is used in the pure form, the product may be continuously extracted in the D-limonene phase. The D-limonene that is not converted may be removed by distillation under low pressure.

It has also been found that the *trans*-carveol product of the present invention may be partially or fully converted to carvone when a specific strain of enzyme is used. Thus, according to another aspect of the present invention there is provided a process for the production of *trans*-carveol and/or carvone which process comprises (a) incubating in the presence of D-limonene, a culture medium comprising *Rhodococcus globerulus* PWD8 (DSMZ 44306) and (b) recovering the carveol and/or carvone from the culture medium.

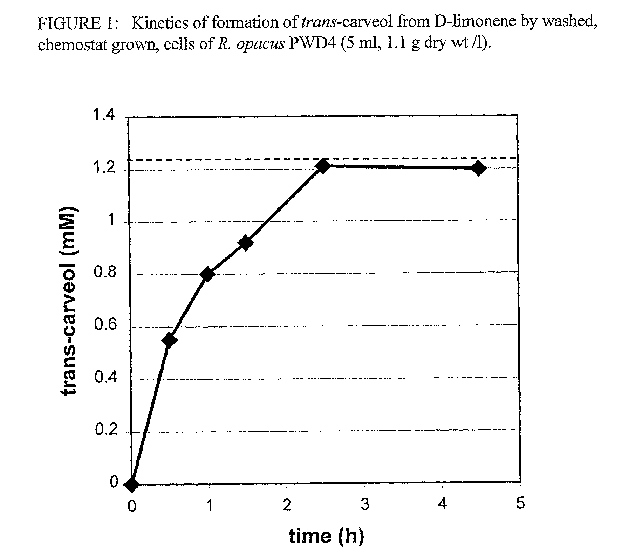

The present invention will now be illustrated with reference to the following examples and with reference to the accompanying Figure 1:
Figure 1 is a graph of the kinetics of formation of *trans*-carveol from D-limonene in the presence of *Rhodococcus opacus* PWD4.
Figure 2 is a the chromatograms from the HPLC analysis of carveol

### Materials and Methods

(1) Screening: Multiple strains stored in one individual 96 well microtiter plate at minus 80 °C were sampled simultaneously (without thawing the remaining culture) using a spring-loaded 96-pin replicator (Kuhner, Basel, Switzerland) as previously described and transferred to a regular sterile polystyrene microtiter plate each well of which (working volume 350 µl) was filled with 180 µl of a solidified agar (2% [w/v]) mineral medium without any C-source. The inoculated microtiter plate (lid on top was kept at a distance of 2 mm from the wells, in order to allow for a sufficient level of gas diffusion in and out the wells) was placed in a dessicator together with a beaker of water and a 50 ml beaker containing 10 ml of a 10% solution (v/v) of toluene in hexadecane.

After 7 days of growth at ambient temperature (22-25 °C) the cell mass developed on the agar surface was harvested as follows. 110 µl of a K₂HPO₄/KH₂PO₄-buffer (50 mM, pH 7.0) was added to each well. Repeated lateral movement of the spring-loaded replicator in the wells resulted in the suspension of a large part of the cell mass. The suspensions were subsequently transferred (using a 12 channel multipipette and wide orifice tips) to a microtiter plate with 0.5 ml conical wells (Maxi-plaque, Polylabo, Geneva). The microtiter plate was centrifuged for 15 min at 4000 rpm in a centrifuge (Eppendorf, type 5403). After disposal of the supernatant, the cells were resuspended in 100 µl of a K₂HPO₄/KH₂PO₄-buffer (50 mM, pH 7.0) by repeated filling and emptying of wide-orifice pipette tips (using a 12 channel multipipette). Subsequently, 2 µl of optically pure D-limonene (Fluka, p.a.) was added to each well and the wells were closed using a sandwich cover (a pierced layer of soft silicone in combination with a rigid polypropylene plate) as described previously (12), followed by 2 hours of orbital shaking at 300 rpm, 5 cm shaking amplitude, at 25 °C. The microtiter plate was then centrifuged for 15 min at 4000 rpm. 50 µl of the supernatant (without the remaining D-limonene phase) was transferred into a half-area microtiter plate (Costar type 3696, Corning, NY) using a 12 channel multipipette.

(2) Analytical Methods: The products were analysed by HPLC and GC. The HPLC (Agilent 1100 series) was equipped with a diode array detector (set at 190 nm) and a mass detector (Agilent) in series. The GC (Varian 3400) was equipped with a flame-inonisation detector (FID).

The results are given in Table 1 below. The last column of the table indicates the type of toluene biodegradation pathway harboured by the indicated strains. It can be seen from the results that the bacterial strains expressing oxygenases capable of dioxygenation of toluene are able to convert D-limonene to *trans*-carveol whilst the other strains are unable to convert D-limonene to *trans*-carveol.

**Table 1:**

| 38 Toluene-degrading strains subdivided according to the concentration of *trans-* carveol formed during a two-hour incubation with D-limonene. | | | |
|---|---|---|---|
| [*Trans*-carveol] ( mM) | Taxonomy | strain codes | pathway |
| 500-1600 µM | *Rhodococcus opacus* | PWD4^{a)}, PWD7^{a)}, PWD10^{a,h)}, PWD19^{a,h)} | tod^{c)} |
| | *Rhodococcus globerulus* | PWD6^{a,g)}, PWD8^{a,g)}, PWD30^{a,h)} | tod^{c)} |
| | *Rhodococcus erythropolis* | PWD2^{a,d)}, PWD3^{a,d)} | tod^{c)} |
| | *Pseudomonas syringae* | PWD16^{b)} | tod^{c)} |
| | *Pseudomonas* sp. | PWD22 | tod^{c)} |
| | *Alcaligenes* sp. | PWD38^{h)} | tod^{c)} |
| 200-380 µM | *Pseudomonas marginalis* | PWD17^{b)}, PWD18^{b)}, PWD26^{b)}, PWD34^{b)} | tod^{c)} |
| | *Pseudomonas syringae* | PWD23^{b)}, PWD31^{b)} | tod^{c)} |
| | *Rhodococcus globerulus* | PWD1^{a)}, PWD28^{a,g)} | tod^{c)} |
| | *Pseudomonas fluorescens* | PWD37^{b)} | tod^{c)} |
| | *Pseudomonas viridiflava* | PWD12^{b)} | tod^{c)} |
| | *Pseudomonas stutzeri* | PWD32^{b)} | tod^{c)} |
| 50-180 µM | *Alcaligenes sp.* | PWD24^{b)} | tod^{c)} |
| | *Pseudomonas syringae* | PWD36^{b)} | tod^{c)} |
| | *Rhodococcus opacus* | PWD15^{a)} | tod^{c)} |
| below detection limit | *Pseudomonas putida* | GJ40, PWD33^{b)} | tod^{c)} |
| | *Pseudomonas marginalis* | PWD27^{b)} | tod^{c)} |
| | *Pseudomonas putida* | mt-2, | TOL^{d)} |
| | *Pseudomonas syringae* | PWD11^{b)}, PWD13^{b)} | TOL^{d)} |
| | *Pseudomonas azotoformans* | PWD20^{b)}, PWD39^{b)} | TOL^{d)} |
| | *Pseudomonas stutzeri* | PWD21^{b)}, PWD40^{b)} | TOL^{d)} |
| | *Pseudomonas marginalis* | PWD35^{b)} | TOL^{d)} |
| | *Pseudomonas mendocina* | KR-1 | KR1^{e)} |
| | *Burkholderia cepacia* | G4 | G4^{f)} |
| a) 100 % 16S RNA gene sequence identity with type strain | | | |
| b) 99.0-99.8% 16S RNA gene sequence identity with type strain | | | |
| c) toluene degradation pathway involving dihydroxylation of the aromatic ring | | | |
| d) toluene degradation pathway involving monohydroxylation of the methyl group | | | |
| e) toluene degradation pathway involving monohydroxylation in the *para*-position (33) | | | |
| f) toluene degradation pathway involving monohydroxylation in the *ortho*-position (28) | | | |
| g) Co-producing carvone in concentrations of 8-16 % of the *trans*-carveol concentrations | | | |
| h) Co-producingcarvone in concentrations of 1-3 % of the *trans*-carveol concentrations | | | |

### Example 1: Production of trans-Carveol using Rhodococcus opacus PWD4:

### 1.1. Growth of Rhodococcus opacus PWD4:

*Rhodococcus opacus* was grown on a mineral medium supplied with 5 mM glucose in an 100 ml Erlenmeyer shaped chemostat at a dilution rate of 0.03 h⁻¹. Toluene was supplied as an additional C-source via the gas phase as described previously, allowing the cells to reach a dry weight of approximately 1 g dry wt per liter.

### 1.2. Production of trans-carveol using Rhodococcus opacus PWD4:

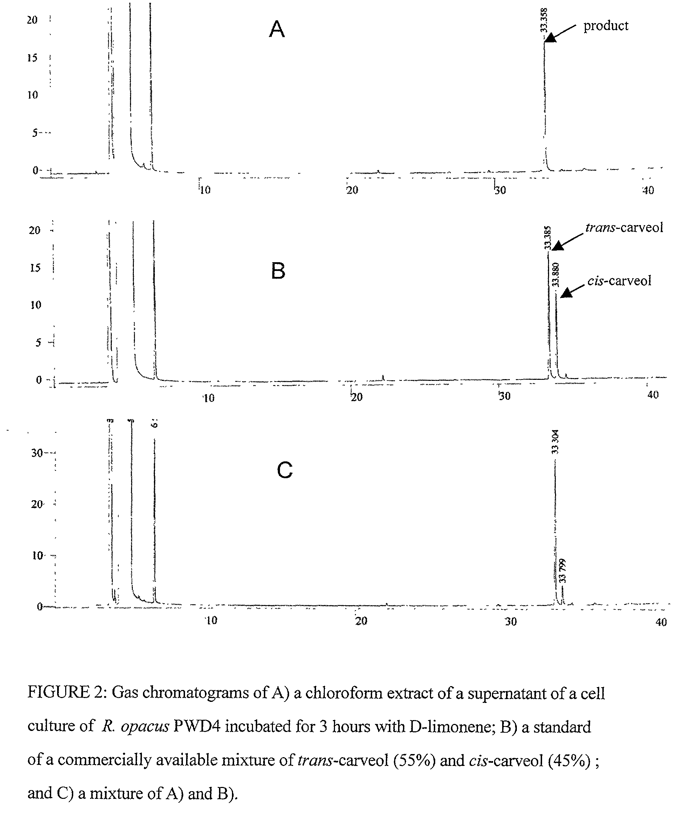

*Rhodococcus opacus* PWD4 was cultivated in chemostat culture at a dilution rate of 0.03 h⁻¹ on a mineral medium with toluene, supplied via the gas-phase, as the main C-source. After the culture had reached a constant dry weight of 1.25 g dry wt l⁻¹, cells were harvested and used to study the biotransformation kinetics of D-limonene in the presence and absence of toluene In the absence of toluene, the maximal concentration of *trans*-carveol was measured after 2 hours (1.21 mM) (Figures 1 and 2).. The only other oxidation product detected was carvone (∼ 15 µM, or 1.3 % of the *trans*-carveol formed). The molar yield of *trans*-carveol in the experiment without toluene was approximately 97% (mol/mol) if the standard of carveol is assumed to be 100% pure, or 94% (mol/mol) if the standard of carveol is only 97% (indicated as the minimum purity by the supplier). A gas chromatogram of a chloroform extract of this biotransformation showed only one significant peak, corresponding exactly to the retention time of *trans*-carveol (Figure 2). The initial specific activity was 14.7 U (g cell dry wt)⁻¹.
The experiment was done in a closed headspace vial with a total volume of 43 ml. The dashed line indicates the theoretical concentration of *trans*-carveol if the molar yield on D-limonene were 100%.

In the presence of toluene, the specific acitivity of *trans*-carveol formation was 0.8 U (g cell dry wt)⁻¹. The average aqueous toluene depletion rate in the first hour of incubation was 22 µM min⁻¹, corresponding to an specific acitivity of toluene consumption (after correction for the toluene [68% of total amount] present in the gaseous form in the headspace) of 58 U (g cell dry wt)⁻¹. After all toluene was depleted, the specific *trans*-carveol formation rate increased 9-fold to 7.8 U (g cell dry wt)⁻¹. Comparison of the maximal toluene degradation rates with the maximal *trans*-carveol formation rates measured indicates that the relative activity on D-limonene was 25 %. These results show that toluene is a reversible competitive inhibitor of the biotransformation of D-limonene to *trans*-carveol.

### Example 2: Co-Production of trans Carveol and carvone using Rhodococcus globerulus PWD8:

Toluene-induced cells of the strain, *Rhodococcus globerulus* PWD8, were incubated with D-limonene. The initial *trans*-carveol concentration (average of the first hour) was 0.73 mM h⁻¹ corresponding to a specific activity of 3 U (g dry wt)⁻¹. After two hours, more than 90% of D-limonene added was transformed to *trans*-carveol (1.1 mM) and a concentration of 0.08 mM carvone was formed. The concentration of carvone gradually increased to maximally 0.29 mM after 27 hrs, while the *trans*-carveol concentration decreased accordingly, indicating that the *trans*-carveol formed was converted slowly to carvone.

## Claims

1. A process for the production of *trans*-carveol which process comprises (a) incubating in the presence of D-limonene, a culture medium comprising microbial cells expressing an oxygenase capable of dioxygenation of toluene and/or napththalene and (b) recovering the *trans*-carveol from the culture medium.

2. A process as claimed in claim 1 in which the microbial cells are fungal, bacterial or yeast.

3. A process as claimed in claim 3 in which the cells are bacterial.

4. A process as claimed in any one of the preceding claims in which the cells expressing oxygenase is selected from strains from the genus *Rhodococcus* and from the genus *Pseudomonas*.

5. A process as claimed in claim 4 in which the strain belongs to the genus *Rhodococcus*

6. A process as claimed in claim 3 in which the *Rhodococcus* is *Rhodococcus opacus* PWD4.

7. A process as claimed in any one of the preceding claims in which the gene encoding the oxygenase is cloned and expressed in a bacterial or yeast strain.

8. A process as claimed in any one of the preceding claims in which the D-limonene is present in the culture medium in the gas phase or in the liquid phase.

9. A process as claimed in any one of the preceding claims in which the incubation is carried out a temperature of from 15 to 40°C, preferably from 20 to 30°C.

10. A process for the production of *trans*-carveol and/or carvone which process comprises (a) incubating in the presence of limonene, a culture medium comprising *Rhodococcus globerulus* PWD8 and (b) recovering the carveol and/or carvone from the culture medium.

11. A process as claimed in claimed 10 in which the *trans*-carveol formed is converted to carvone
